# FASCICULE DE BREVET EUROPEEN

(11) **EP 3 624 702 B1**
(45) Date de publication et mention de la délivrance du brevet: **02.06.2021**
(21) Numéro de dépôt: 18724977.6
(22) Date de dépôt: 25.04.2018
(51) Int. Cl.: A61B 17/06

(54) **PROCEDE DE FABRICATION D'UN FIL CHIRURGICAL A PICOTS**
VERFAHREN ZUR HERSTELLUNG EINES CHIRURGISCHEN FADENS MIT HAKEN
METHOD FOR THE MANUFACTURE OF A SURGICAL THREAD WITH BARBS

(30) Priorité: 19.05.2017 FR 1754445
(43) Date de publication de la demande: 25.03.2020
(73) Titulaire: JSQ53, 54000 Nancy (FR)
(72) Inventeur: FRISMAND, Jean, 59700 Marc En Baroeul (FR)
(74) Mandataire: Bureau Duthoit Legros Associés
(86) Numéro de dépôt international: PCT/FR2018/051051
(87) Numéro de publication internationale: WO 2018/211193

(56) Documents cités:
- EP-A1- 2 517 633
- EP-A1- 2 690 206
- WO-A2-2009/129251
- DE-A1-102010 036 979
- US-A1- 2010 023 055
- US-A1- 2012 130 406
- US-A1- 2012 150 194
- US-A1- 2012 296 345
- US-A1- 2015 094 760

## Description

La présente invention concerne un procédé de fabrication d'une portion de fil chirurgical à picots.

Le domaine de l'invention est celui des fils chirurgicaux à picots, employés notamment pour la reconstruction dans le domaine de la chirurgie, et/ou de la médecine esthétique dans le traitement contre le vieillissement des tissus ou les problèmes de ptose.

Les fils chirurgicaux comportant des moyens d'accrochage sont bien connus pour la reconstruction dans le domaine de la chirurgie, et/ou de la médecine esthétique et sont utilisés depuis de nombreuses années dans le traitement contre le vieillissement des tissus ou les problèmes de ptose. De tels fils sont généralement réalisés en matériaux polymère et sont pourvus de moyens d'accrochage en saillie depuis la surface périphérique du fil et répartis sur l'ensemble de cette surface périphérique.

De façon bien connue, les moyens d'accrochage peuvent consister en des picots, encore appelées « *barbes* », des bourrelets, des nœuds, des cônes, etc. et peuvent être ménagés d'un seul tenant avec le fil ou consister en des éléments rapportés.

De tels fils chirurgicaux sont ainsi implantés sous la peau d'un patient et les moyens d'accrochage viennent se fixer aux tissus de la partie du visage ou du corps à traiter afin d'exercer une tension sur ces derniers pour les soutenir.

Selon les matériaux composant le fil chirurgical, la forme et la disposition des moyens d'accrochage, différents procédés de fabrication de ces fils chirurgicaux peuvent être employés.

On connaît par exemple du document WO 2008/112417 A2 un procédé de fabrication d'un fil chirurgical présentant des moyens d'accrochage, le fil comportant un noyau réalisé dans un premier matériau, ayant une certaine rigidité, et une gaine entourant ce noyau, comprenant au moins une crête, la gaine étant réalisée dans un second matériau, ayant une certaine rigidité, différente, notamment supérieure, de celle du premier matériau, les moyens d'accrochage étant réalisés dans la gaine, au niveau de la au moins une crête.

Le procédé de fabrication d'un tel fil chirurgical comporte les étapes suivantes :
- fabrication par coextrusion, à partir de deux matériaux polymères différents, d'un fil brut comprenant un noyau dans le premier matériau recouvert d'une gaine dans le second matériau, la coextrusion se faisant au travers d'une filière ayant une section telle que la gaine comporte au moins une crête,
- refroidissement du fil brut,
- étirage du fil afin de créer un fil orienté en le faisant passer à travers une série de rouleaux afin de multiplier sa longueur par un nombre compris entre deux et dix,
- formation des moyens d'accrochage par enlèvement de matière au niveau de la au moins une crête de la gaine.

Comme compréhensibles des exemples de réalisation 1 et 2, donnés dans le document WO 2008/112417 A2, le procédé divulgué dans ce document prévoit que l'étape de formation des moyens d'accrochage soit réalisée après l'étape d'étirage du fil.

Un tel fil chirurgical présente l'inconvénient d'avoir des moyens d'accrochage peu résistants, car l'enlèvement de matière nécessaire à leur formation fragilise le fil chirurgical, notamment la gaine dans laquelle sont formés ces moyens d'accrochage, en créant une amorce de rupture. Les moyens d'accrochage risquent ainsi de se détacher du fil lors de la pose du fil ou peu de temps après, ce qui peut s'avérer dangereux pour le patient.

De plus, la finesse des moyens d'accrochage obtenus par enlèvement de matière risque de les voir se retourner lors de la pose du fil sur le patient, les empêchant de s'accrocher, diminuant ou annulant l'efficacité du fil chirurgical.

Enfin, la nécessité d'avoir un fil chirurgical composé d'un noyau dans un premier matériau et d'une gaine dans un second matériau, dans laquelle sont réalisés les moyens d'accrochage, permet certes d'obtenir un fil chirurgical avec une rigidité bien maîtrisée mais ne permet en revanche pas d'obtenir un fil fin, notamment de diamètre inférieur à 0,5mm, ce qui nuit à la capacité du fil chirurgical à pouvoir être posé à différents endroits du corps d'un patient, complexifie sa pose sur un patient et peut créer une sensation d'inconfort pour le patient après la pose.

On connaît également du document EP 2 690 206 A1 un procédé de fabrication d'un fil chirurgical comprenant deux étapes :
(1) formation de micro-dents à la surface du fil surface par chauffage et pressage du matériau brut du fil chirurgical dans un moule à débordement. Comme visible par exemple sur la figure 1 de ce document, les micro-dents sont formées au niveau du plan de joint uniquement, à savoir dans un seul plan. Comme visibles sur les figures 1 et 2 et comme expliqué par exemple aux paragraphes [0037] et [0045] de ce document, à l'issue de l'étape (1), les micro-dents sont massives, en ce qu'elles ont un débord inférieur au diamètre du fil et le diamètre de leur base est au moins égal au diamètre du fil.
(2) En sortie du moule, un effort de traction, compris entre 10% et 30% de l'effort de rupture à la traction du fil est appliqué pendant une longue durée (entre 24h et 48h), en même temps qu'un effort de torsion, permettant de positionner les micro-dents selon une disposition hélicoïdale à la surface du fil, le fil étant également chauffé au cours de cette étape.

Un fil chirurgical obtenu par un tel procédé présente plusieurs inconvénients. Premièrement, le fait d'appliquer un effort de traction et de torsion au fil, permet certes une réorientation des molécules de polymère dans le matériau afin d'améliorer sa résistance à la traction, mais la durée importante d'application va contrebalancer cet effet et fragiliser le fil, qui aura à l'issue du procédé une résistance à la traction dégradée, et donc une durée de vie moindre.

De plus, cette durée importante de l'étape (2) va considérablement augmenter la durée totale du procédé de fabrication d'un tel fil.

Par ailleurs, les micro-dents du fil étant massives, elles pénètreront mal dans les tissus du patient, ce qui réduit sensiblement l'efficacité d'un tel fil chirurgical, mais peut également créer une gêne pour le patient.

On connaît également les documents US2010/023055 A1, US2012/130406 A1 US2015/094760 A1 et WO2009/129251 A2 qui décrivent aussi des procédés de fabrication de fils chirurgicaux.

L'objectif de la présente invention est de pallier les inconvénients précités en proposant un procédé de fabrication d'une portion de fil chirurgical munie de moyens d'accrochage permettant d'obtenir une portion de fil chirurgical avec une résistance, notamment à la traction, améliorée.

Un autre but de la présente invention est de proposer un procédé de fabrication d'une portion de fil chirurgical dont les moyens d'accrochage ne risquent pas de se retourner ou de se détacher pendant ou après l'implantation du fil chirurgical sous la peau d'un patient.

Un autre but de la présente invention est de proposer un procédé de fabrication d'une portion de fil chirurgical permettant d'obtenir une portion de fil chirurgical facile à poser sur un patient et dont les picots ne risquent pas d'arracher les tissus auxquels ils seront accrochés et de créer une douleur chez le patient.

Enfin, un autre but de l'invention est de proposer un procédé rapide et simple à mettre en œuvre, et permettant d'obtenir une portion de fil chirurgical à picots au coût de revient peu élevé.

Ainsi, l'invention concerne un procédé de fabrication d'une portion de fil chirurgical pour la reconstruction dans le domaine de la chirurgie, et/ou de la médecine esthétique, ladite portion de fil chirurgical comprenant une partie de support, de forme cylindrique, et une pluralité de picots saillants de la partie de support et répartis de façon régulière sur la partie de support, ladite portion de fil chirurgical ayant une longueur Lf, ledit procédé comprenant les étapes successives :
a). Obtention d'une portion de fil brute de longueur Li, avec Li<Lf, munie de picots réalisés par moulage dans un moule à picots comprenant une pluralité d'empreintes permettant la formation des picots,
b). Etirage de la portion de fil brute, la portion de fil chirurgical ayant à l'issue de cette étape la longueur Lf.

Selon l'invention, au cours de l'étape b), l'étirage de la portion de fil brute (10) est tel que sa longueur Li est multipliée au moins par 2.

Selon un mode de réalisation :
- à l'issue de l'étape a), lesdits picots sont inclinés d'un angle ai par rapport à l'axe longitudinal de la portion de fil brute, l'angle ai étant compris entre 60° et 90°, de préférence entre 70° et 80°, et
- à l'issue de l'étape b), sous l'effet de l'étirage de la portion de fil brute, les picots se retrouvent inclinés d'un angle af par rapport à l'axe longitudinal de la portion de fil chirurgical, l'angle af étant strictement inférieur à l'angle ai et étant comprise entre 30° et 60°, de préférence entre 40° et 50°.

Selon un mode de réalisation la longueur des picots est supérieure au diamètre de la portion de fil chirurgical, et la largeur de leur base, ménagée sur la partie de support de la portion de fil chirurgical, est inférieure au diamètre, de la portion de fil chirurgical.

Selon un mode de réalisation, l'étape a) d'obtention d'une portion de fil brute comprend au moins trois sous-étapes a1), a2) et a3) :
a1). Formation par extrusion de la partie de support de la portion de fil brute à partir d'au moins un matériau polymère brut, à l'issue de laquelle la portion de fil brute possède la longueur (Li),
a2). Introduction de la partie de support de la portion de fil brute dans un moule à picots,
a3). Mise sous pression de la partie de support de la portion de fil brute dans le moule à picots afin de former les picots saillants de la partie de support, lesdits picots étant, à l'issue de cette étape, inclinés par rapport à l'axe longitudinal de l'angle ai.

Selon un mode de réalisation, l'étape a) d'obtention d'une portion de fil brute comprend une unique sous-étape a1bis) de moulage par injection dans un moule à picots de la partie de support de la portion de fil brute et des picots saillants de la partie de support à partir d'au moins un matériau polymère brute.

Selon un mode de réalisation, les picots et la partie de support sont réalisés dans le même matériau polymère.

Selon un mode de réalisation, une étape c) de chauffage de la portion de fil brute munie de picots est prévue après l'étape a), et avant ou simultanément à l'étape b).

Selon un mode de réalisation, les picots comportent une extrémité de forme hémisphérique avec un rayon de courbure supérieur à 0,1 mm.

Selon un mode de réalisation, la portion de fil chirurgical est résorbable et comprend au moins un matériau polymère résorbable.

Selon un mode de réalisation, la portion de fil chirurgical est constituée d'un mélange de PLLA et de PCL, notamment un mélange de 60% à 95% de PLLA et de 5% à 40% de PCL.

Selon un mode de réalisation, les picots sont répartis sur la partie de support de la portion de fil chirurgical et de la portion de fil brute selon au moins quatre rangées, orientées parallèlement à l'axe longitudinal de la portion de fil chirurgical et de la portion de fil brute et disposées de façon régulière tout autour de l'axe longitudinal de la portion de fil chirurgical et de la portion de fil brute.

Selon un mode de réalisation, le moule à picots employé au cours de l'étape a) est conformé de telle sorte qu'il comporte au moins quatre parties, notamment identiques, ces parties étant séparées par des évents, lorsque le moule est fermé et mis sous pression, ces évents étant disposés au niveau des plans de symétrie de la portion de fil brute munie de picots, séparant chacun des picots en deux parties symétriques par rapport à un de ces plans de symétrie, et débouchant dans les empreintes permettant la formation des picots.

On décrit également un fil chirurgical pour la reconstruction dans le domaine de la chirurgie, et/ou de la médecine esthétique comprenant au moins une portion réalisée par le procédé selon l'invention.

L'invention sera mieux comprise à la lecture de la description suivante accompagnée des dessins parmi lesquels :
- La figure 1 est une vue schématique de côté d'une portion de fil chirurgical obtenu grâce au procédé selon l'invention,
- La figure 2 est une vue schématique de côté de la partie de support de la portion de fil brute,
- La figure 3 est une vue schématique de côté de la portion de fil brute obtenue à l'issue de l'étape a) du procédé selon l'invention,
- La figure 4a est un diagramme représentant le procédé selon l'invention,
- La figure 4b est un diagramme représentant une première variante de l'étape a) du procédé selon l'invention,
- La figure 4c est un diagramme représentant une deuxième variante de l'étape a) du procédé selon l'invention,
- La figure 5 est une vue schématique représentant une installation permettant de mettre en œuvre le procédé selon l'invention,
- La figure 6 est une vue schématique représentant une deuxième variante d'une installation permettant de mettre en œuvre le procédé selon l'invention,
- La figure 7 est une vue en coupe selon la ligne IX-IX de la figure 1, représentant schématiquement une section de la portion de fil chirurgical 1,
- La figure 8 est une vue schématique représentant un moule à picots permettant de mettre en œuvre l'étape a du procédé selon l'invention.

Aussi l'invention concerne un procédé de fabrication d'une portion de fil chirurgical 1 pour la reconstruction dans le domaine de la chirurgie, et/ou de la médecine esthétique, dans lequel ladite portion de fil chirurgical 1 comprend une partie de support 2, de forme cylindrique, et une pluralité de picots 3 saillants de la partie de support 2 et répartis de préférence de façon régulière sur la partie de support 2, ladite portion de fil chirurgical 1 ayant une longueur Lf, ledit procédé comprenant les étapes successives :
a). Obtention d'une portion de fil brute 10 de longueur Li, avec Li<Lf, munie de picots 3 réalisés par moulage dans un moule à picots M comprenant une pluralité d'empreintes EP permettant la formation des picots 3,
b). Etirage de la portion de fil brute 10, la portion de fil chirurgical 1 ayant à l'issue de cette étape la longueur Lf.

Selon l'invention, au cours de l'étape b), l'étirage de la portion de fil brute 10 est tel que sa longueur Li est multipliée au moins par 2.

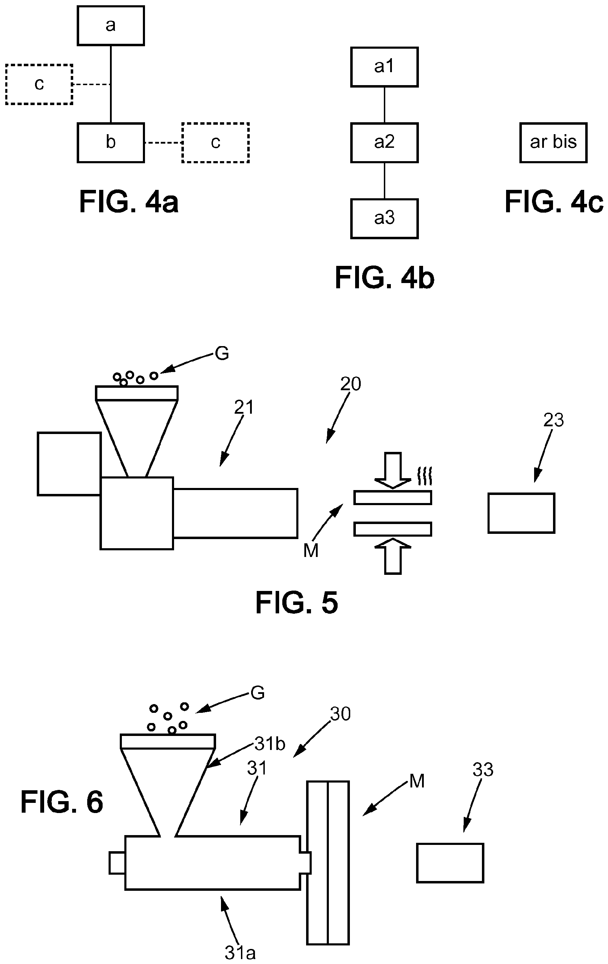

Ce procédé est représenté schématiquement sur l'exemple de réalisation de la figure 4a.

Avantageusement, la portion de fil chirurgical 1 est prévue pour être réalisée dans un matériau polymère, notamment un matériau polymère biocompatible, constitué éventuellement d'un mélange de plusieurs matériaux polymères.

Les picots 3 saillants de la partie de support 2 étant réalisés par moulage sur la partie de support, leur fixation à la partie de support 2 de la portion de fil chirurgical 1 ne sera pas fragilisée par une amorce de rupture créée par un enlèvement de matière.

De plus, une portion de fil chirurgical 1 obtenue par un tel procédé présentera une excellente résistance à la traction, avec une résistance à la rupture en traction typiquement de l'ordre de 20N pour un diamètre de la partie de support de 0.5 mm, soit environs 100 MPa.

En effet, bien que le chauffage et la mise sous pression de la portion de fil brute 10 au cours de l'étape a) aient pu la fragiliser, notamment en réduisant sa résistance à la traction, l'étirage au cours de l'étape b) de cette portion de fil brute 10 permet de déformer plastiquement la portion de fil brute 10, et notamment la partie de support 2, ce qui entraîne une réorientation des molécules du matériau composant la partie de support 2 de la portion de fil chirurgical 1 de sorte à améliorer considérablement la résistance mécanique en traction de la portion de fil chirurgical 1.

Avantageusement, cette étape b peut consister en l'application pendant une durée brève, notamment inférieure à 1 minute, d'un effort de traction, notamment strictement inférieur à l'effort de rupture en traction de la portion de fil brute 10, apte à déformer la portion de fil brute 10 plastiquement. Avantageusement, l'étape b) d'étirage doit être prévue de sorte à multiplier la longueur de la portion de fil brute par au moins deux, afin d'obtenir une résistance en traction satisfaisante, mais ne doit par exemple pas dépasser une multiplication par dix, afin de ne pas trop affiner la portion de fil chirurgical 1, ce qui nuirait à sa résistance en traction à cause d'une section trop faible. Un bon compromis permettant d'obtenir une résistance en traction satisfaisante et une section de la partie de support 2 suffisante peut être de multiplier la longueur Li de la portion de fil brute 10 entre quatre et six fois avec un diamètre Df de la partie de support 2 de la portion de fil chirurgical 1 compris entre 0,1 mm et 1 mm, par exemple 0,6 mm. Un tel diamètre Df de la partie de support 2 de la portion de fil chirurgical 1 est néanmoins suffisamment fin pour faciliter la manipulation de la portion de fil chirurgical 1 lors de la pose sur un patient.

Une portion de fil chirurgical 1 obtenue par un tel procédé présente ainsi une partie de support 2 de forme cylindrique, prévue pour assurer la rigidité et l'élasticité de la portion de fil chirurgical 1.

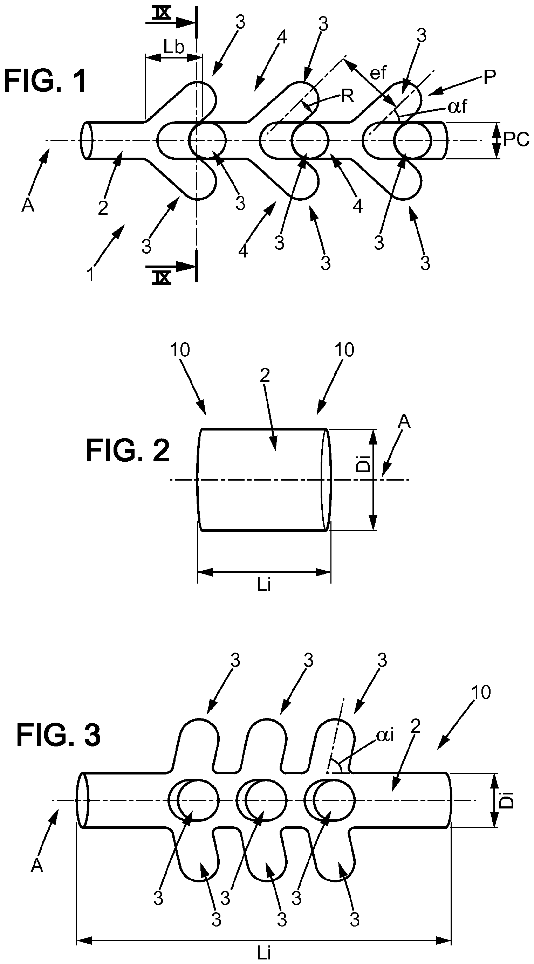

Comme représenté sur l'exemple de réalisation des figures 1 à 3, une telle partie de support 2 est généralement de section circulaire mais toute autre forme géométrique, simple ou complexe peut être envisagée.

Comme représenté sur l'exemple de réalisation des figures 1, 3 et 8, les picots 3 sont avantageusement des éléments de forme globalement cylindrique ou éventuellement conique de section circulaire, ou alternativement de section de toute autre forme géométrique simple ou complexe, leur base se trouvant ménagée sur la partie de support 2.

Comme représenté sur l'exemple de réalisation de la figure 1 et de la figure 2, les picots 3 peuvent être répartis régulièrement sur l'ensemble de la partie de support 2 de la portion de fil chirurgical 1 et de la portion de fil brute 10. Par répartis régulièrement, on entend que les picots 3 sont répartis selon plusieurs rangées 4, notamment quatre, autour de l'axe longitudinal A de la portion de fil chirurgical 1 et de la portion de fil brute 10, chaque rangée 4 étant constituée de picots 3 alignés selon un axe, notamment de direction, parallèle à l'axe longitudinal A, et deux picots 3 successifs d'une même rangée 4 étant séparés d'une distance ef, notamment identique sur toutes les rangées 4 de picots 3, par exemple comprise entre 1 et 2 mm, notamment égale à 1,5 mm, sur toute la rangée.

Les rangées 4 de picots 3 sont prévues pour se retrouver dans leur position définitive à l'issue de l'étape a) du procédé selon l'invention. Ainsi, aucune déformation en torsion de la portion de fil brute 10 ne sera nécessaire au cours du procédé selon l'invention afin de positionner les picots 3 tout autour de la partie de support 2 de la portion de fil chirurgical 1.

De manière notable et selon un mode de réalisation avantageux de l'invention :
- à l'issue de l'étape a), lesdits picots 3 sont inclinés d'un angle ai par rapport à l'axe longitudinal A de la portion de fil brute 10, l'angle ai étant compris entre 60° et 90°, de préférence entre 70° et 80°, et
- à l'issue de l'étape b), sous l'effet de l'étirage de la portion de fil brute 10, les picots 3 se retrouvent inclinés d'un angle af par rapport à l'axe longitudinal A de la portion de fil chirurgical 1, l'angle af étant strictement inférieur à l'angle ai et étant compris entre 30° et 60°, de préférence entre 40° et 50°.

Comme représenté sur l'exemple de réalisation de la figure 1, les picots 3 sont prévus pour être inclinés par rapport à l'axe longitudinal A de la portion de fil chirurgical selon un angle af compris entre 30° et 60°, de préférence entre 40° et 50°. Une telle inclinaison permet, lors de la pose sur un patient, de faciliter le déplacement de la portion de fil chirurgical dans une premier sens, opposé au sens d'inclinaison des picots 3, et de permettre un accrochage optimal de la portion de fil chirurgical 1 sur les tissus du patient à traiter, lorsque l'on exerce une traction sur celle-ci en sens inverse (selon le sens d'inclinaison des picots 3).

L'inventeur a néanmoins constaté, de façon surprenante, que l'étape b) d'étirage provoquait un affaissement des picots réalisés sur la portion de fil brut : le mode de réalisation précité prend avantageusement en compte ce phénomène en choisissant pour l'étape de moulage un moule dont les empreintes EP pour la formation des picots inclinent les picots dudit angle ai à l'issue de l'étape a) de moulage, supérieur l'angle af à l'issue de l'étape b), et afin de tenir compte de la perte d'inclinaison due à l'étape d'étirage b). Autrement dit les picots 3 formés sur la partie de support 2 de la portion de fil brute 10 à l'issue de l'étape a) du procédé selon l'invention, inclinés d'un angle ai par rapport à l'axe longitudinal A de la portion de fil brute 10, se retrouvent, sous l'effet de l'effort de traction exercé sur la portion de fil brute au cours de l'étape b) d'étirage inclinés d'un angle af par rapport à l'axe longitudinal A de la portion de fil chirurgical 1, l'angle af étant strictement inférieur à l'angle ai, et demeuraient selon cette inclinaison à l'issue de l'étape b.

Ainsi, selon la disposition avantageuse de l'invention, le fait de réaliser une portion de fil brute 10, représentée par exemple à la figure 2, comportant des picots 3 inclinés par rapport à l'axe longitudinal A de la portion de fil brute 10 ou à l'axe longitudinal A de la portion de fil chirurgical 1, d'un angle ai strictement supérieur à af, et compris entre 60° et 90°, de préférence entre 70° et 80°, permet de façon avantageuse de s'assurer d'obtenir à l'issue du procédé selon l'invention, et notamment de l'étape b) d'étirage, une portion de fil chirurgical 1 munie de picots 3 inclinés par rapport à l'axe longitudinal selon l'angle af souhaité.

En effet, lors de l'étape b) d'étirage de la portion de fil brute 10, les picots 3 saillants de la partie de support 2, sous l'effet de l'effort de traction appliqué à la partie de support 2, vont s'incliner en se rapprochant de la partie de support 2, et à cause de la déformation plastique de la partie de support 2, demeurer en cette position à l'issue de l'étape b).

Selon l'invention, la longueur Lp des picots 3 est de préférence supérieure au diamètre Df de la portion de fil chirurgical 1, par exemple égale à 1,5 mm, et la largeur Lb de leur base, ménagée sur la partie de support 2 de la portion de fil chirurgical 1, est inférieure au diamètre Df de la portion de fil chirurgical 1, par exemple égale à 0,5 mm.

La longueur Lp des picots 3 correspond à la distance séparant le centre de la base d'un picot 3 au point d'extrémité supérieur du picot 3, représentée par exemple sur l'exemple de réalisation de la figure 1.

La largeur Lb de la base des picots 3, représentée par exemple sur l'exemple de réalisation de la figure 1, correspond à la largeur de la section transversale, notamment circulaire, d'un picot 3 appartenant à la partie de support 2.

Grâce à cette disposition avantageuse, les picots 3 sont longs et fins, notamment par rapport au diamètre Df de la portion de fil chirurgical 1, ce qui favorisera leur pénétration dans les tissus du patient tout en réduisant la sensation de gêne que pourrait ressentir le patient à l'issue de la pose de la portion de fil chirurgical 1.

La propension des picots 3 à diminuer l'angle de leur inclinaison par rapport à l'axe longitudinal A au cours de l'étape b), décrite précédemment, est accentuée lorsque la portion de fil chirurgical 1 est munie de picots 3 fins, tels que décrits précédemment, dont la largeur Lb de la base, ménagée sur la partie de support 2 de la portion de fil chirurgical 1, n'est pas suffisante pour garantir le maintien de leur inclinaison au cours de l'étape b).

Selon l'invention, l'étape a) d'obtention d'une portion de fil brute comprend au moins trois sous-étapes a1), a2) et a3) :
a1). Formation par extrusion de la partie de support de la portion de fil brute à partir d'au moins un matériau polymère brut, à l'issue de laquelle la portion de fil brute possède la longueur Li,
a2). Introduction de la partie de support de la portion de fil brute dans un moule à picots,
a3). Mise sous pression de la partie de support de la portion de fil brute dans le moule à picots afin de former les picots saillants de la partie de support, lesdits picots étant, à l'issue de cette étape, inclinés par rapport à l'axe longitudinal de l'angle ai.

Cette variante de l'étape a) du procédé est représentée schématiquement sur l'exemple de réalisation de la figure 4b.

Cette variante de l'étape a) du procédé selon l'invention peut par exemple être mise en œuvre dans une installation 20 comportant au moins une extrudeuse 21, représentée par exemple à la figure 5.

Lors de l'étape a1), le matériau polymère brut peut par exemple se présenter sous la forme de granulés G du ou des matériaux polymères constituant le matériau polymère de la portion de fil chirurgical 1. Au cours de cette même étape a1), les granulés G peuvent être introduits dans le réservoir d'alimentation d'une extrudeuse 21, afin d'être chauffés et extrudés au travers d'une filière conformée de sorte à obtenir en sortie de l'extrudeuse 21 la partie de support 2 de la portion de fil brute 10, avec la longueur Li, représentée par exemple à la figure 2.

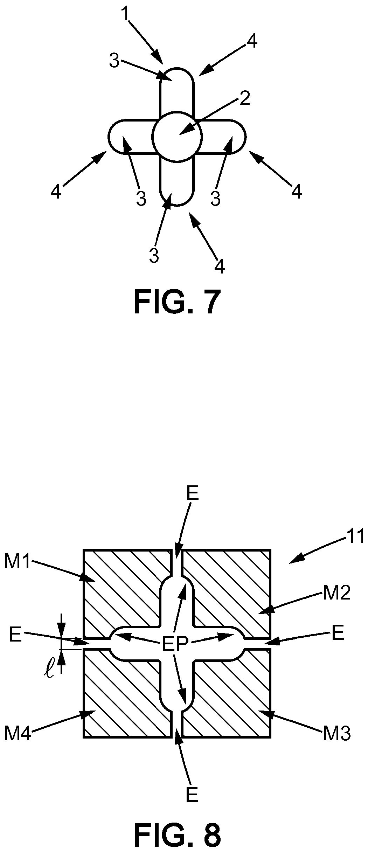

La partie de support 2 de la portion de fil brute ainsi formée peut ensuite être introduite dans un moule à picots M, au cours de l'étape a2. Le moule à picots M, représenté sur l'exemple de réalisation de la figure 7, comporte avantageusement une pluralité d'empreintes de picots EP, disposées selon la disposition des picots 3 sur la partie de support 2 de la portion de fil brute 10 souhaitée.

Au cours de l'étape a3) le moule à picots M, dans lequel la portion de fil brute 10 comportant uniquement la partie de support 2 a été introduite, est mis en pression, par tout moyen connu de l'Homme du métier, par exemple par l'intermédiaire d'un circuit hydraulique. Les picots 3 vont ainsi être formés sur la partie de support 2 par l'intermédiaire des empreintes de picots EP. Après un temps suffisamment long pour que les picots 3 soient correctement formés et refroidis, la portion de fil brute 10 munie de picots 3 peut être extraite du moule à picots M.

Un refroidissement, par exemple au moyen d'un fluide tel que l'eau ou l'air, peut être mis en œuvre pour refroidir le moule contenant la portion de fil brute 10, notamment afin de faciliter sa manipulation par un opérateur, à l'issue de l'étape a3).

Un chauffage peut également être mis en œuvre, postérieurement à l'étape a1) afin de ramollir la portion de fil brute 10 pour faciliter la formation des picots 3 sur la partie de support 2 de la portion de fil brute 10 au cours de l'étape a3).

Selon un mode de réalisation alternatif de l'invention, l'étape a) d'obtention d'une portion de fil brute 10 comprend une unique sous-étape a1bis) de moulage par injection dans un moule à picots M de la partie de support 2 de la portion de fil brute 10 et des picots 3 saillants de la partie de support 2 à partir d'au moins un matériau polymère brute.

Cette variante de l'étape a) du procédé est représentée schématiquement sur l'exemple de réalisation de la figure 4c.

L'étape a1bis) du procédé selon l'invention peut par exemple être mise en œuvre dans une installation 30 comportant au moins un dispositif de moulage par injection 31, par exemple une presse d'injection 31, représentée par exemple à la figure 6.

La presse d'injection 31 comporte de façon classique un réservoir d'alimentation 31b coopérant avec un système d'injection 31a et un moule à picots M. Le moule à picots M, visible sur l'exemple de réalisation de la figure 7, peut par exemple comporter une pluralité d'empreintes de picots EP disposées selon la disposition des picots 3 sur la partie de support 2 de la portion de fil brute 10 souhaitée.

Des granulés G du ou des matériaux polymères constituant le matériau polymère de la portion de fil chirurgical 1 sont par exemple introduits dans le réservoir d'alimentation 31b de la presse d'injection 31, afin d'être chauffés dans le système d'injection de sorte à alimenter le moule à picots M avec du matériau polymère sous forme liquide. Le moule à picots M peut ensuite être pré-chauffé, par exemple par un chauffage à circulation de fluide caloriporteur, et être mis sous pression, par exemple par l'intermédiaire d'un circuit hydraulique. Le matériau polymère sous forme liquide va ainsi remplir les empreintes à picots du moule à picots M et après un temps suffisamment long pour que les picots 3 ainsi que la partie de support 2 soient correctement formés et le moule refroidi, éventuellement au moyen d'un fluide tel que l'eau ou l'air, le moule à picots M peut être ouvert et un dispositif d'éjection (non représenté) peut ensuite être mis en œuvre afin d'éjecter la portion de fil brute 10 munie de picots formé par la presse à injection 31.

L'étape b) d'étirage de la portion de fil brute 10 peut par exemple être mise en œuvre par un dispositif d'étirage 23, 33 prévu dans une installation 20, 30 permettant de mettre en œuvre le procédé selon l'invention.

Le dispositif d'étirage 23, 33 peut consister en tout moyen d'étirage connu de l'Homme du métier, et peut même être réalisé manuellement par un opérateur.

La portion de fil brute 10 va ainsi se déformer plastiquement en traction au cours de l'étape b) et sa longueur va passer de Li à Lf.

Par ailleurs, le diamètre Di de la portion de fil brute 10, tel que Di>Df, par exemple compris entre 1 mm et 2 mm, par exemple égal à 1,5mm, va diminuer de sorte à atteindre la valeur Df.

De même, les picots 3 de la portion de fil brute 10 étant par exemple écartés d'une distance ei, par exemple comprise entre 0,1 et 0,5 mm à l'issue de l'étape a), par exemple égale à 0,2 mm, ceux-ci vont se retrouver écartés de la distance ef à l'issue de l'étape b). L'écartement ei ou ef entre deux picots successifs, représenté par exemple aux exemples de réalisation des figures 1 et 2, correspond à la distance séparant les axes de révolution de deux picots successifs.

Cependant, le procédé selon l'invention, et notamment l'étape b) d'étirage ne va pas avoir d'effet sur les picots 3, autre que la modification de leur inclinaison par rapport à l'axe longitudinal A et leur écartement, l'effort de traction étant exercé sur la partie de support 2. Ainsi, l'étape b) ne va pas fragiliser les picots 3 obtenus à l'étape a) en les déformant plastiquement, ce qui permet d'obtenir une portion de fil chirurgical 1 avec des picots 3 particulièrement résistants, notamment par rapport à des moyens d'accrochage obtenus par enlèvement de matière.

Le dispositif d'étirage 23, 33 est avantageusement indépendant du dispositif 21, 31 de mise en œuvre de l'étape a du procédé selon l'invention. Par exemple, sur les exemples de réalisation des figures 5 et 6, les dispositifs d'étirage 23, 33, peuvent être identiques.

Selon l'invention, les picots 3 et la partie de support 2 sont réalisés de préférence dans le même matériau polymère.

Grâce à cette disposition avantageuse, les picots 3 ne risquent pas de se désolidariser facilement de la partie de support 2, ce qui peut être le cas lorsque les picots 3 et la partie de support 2 sont dans des matériaux distincts, par exemple lorsque les picots 3 sont surmoulés sur la partie de support 2.

Selon l'invention, une étape c) de chauffage de la portion de fil brute 10 munie de picots 3 est prévue après l'étape a), et avant ou simultanément à l'étape b).

Cette variante de l'étape a) du procédé est représentée schématiquement sur l'exemple de réalisation de la figure 4a.

Une telle étape de chauffage c) peut être mise en œuvre par l'intermédiaire d'un dispositif de chauffage (non représenté), comprenant par exemple un fluide, tel que de l'eau ou de l'air, chauffé à une température élevée. Le dispositif de chauffage peut être prévu en amont du dispositif d'étirage 23, 33. Cette étape c) de chauffage permet de faciliter la déformation plastique de la portion de fil chirurgical 1 au cours de l'étape b) d'étirage.

Selon un mode de réalisation de l'invention, les picots 3 comportent une extrémité P de forme hémisphérique avec un rayon de courbure supérieur à 0,1 mm.

Comme visible sur l'exemple de réalisation de la figure 1, les picots 3 sont munis d'une extrémité P permettant leur pénétration dans les tissus du patient. Cependant, afin de ne pas exercer un effort de pénétration trop important sur ces tissus, ce qui pourrait créer un déchirement non souhaité de ceux-ci et entraîner des douleurs chez le patient, il est prévu que les picots 3 aient une extrémité P « arrondie ». L'extrémité P des picots 3 est ainsi de forme hémisphérique et afin d'avoir une capacité de pénétration dans les tissus suffisante avec un effort de pénétration acceptable, le rayon de courbure de cette extrémité P est supérieur à 0,1mm, et peut éventuellement être avantageusement inférieur à 1mm, par exemple égal à 0,25 mm.

Selon l'invention, la portion de fil chirurgical 1 est résorbable et comprend à cet effet au moins un matériau polymère résorbable.

Un matériau polymère résorbable est un matériau biodégradable qui va se dissoudre petit à petit au contact des tissus du patient avec lesquels il sera en contact après la pose de la portion de fil chirurgical 1 sur le patient.

Cette disposition avantageuse de l'invention permet de limiter la durée de vie de la portion de fil chirurgical 1 après la pose sur le patient, afin de diminuer les risques de complications des tissus en contact avec la portion de fil chirurgical 1 dus à un temps de séjour trop long de celle-ci sur le patient.

Par ailleurs, cette disposition avantageuse de l'invention permet également d'éviter une opération supplémentaire sur le patient pour enlever la portion de fil chirurgical 1 après sa pose sur le patient.

Selon l'invention, la portion de fil chirurgical 1 est constituée d'un mélange de PLLA et de PCL, notamment un mélange (intime) de 60% à 95% de PLLA et de 5% à 40% de PCL, la somme de PLLA et de PCL faisant 100%. L'acide Poly (L)-lactique (PLLA) et la Polycaprolactone (PCL) sont des matériaux polymères biodégradables particulièrement adaptés à la mise en œuvre de la présente invention, notamment lorsque la portion de fil chirurgical 1 est constituée d'un mélange de 70% de PLLA et de 30% de PCL.

En effet, en plus de leur biodégradabilité, ces matériaux présentent une bonne moulabilité facilitant le moulage au cours de l'étape a) du procédé selon l'invention ainsi que des résistances à la rupture en traction offrant une bonne résistance à la rupture en traction à la portion de fil chirurgical 1.

Selon l'invention, les picots 3 sont répartis sur la partie de support 2 de la portion de fil chirurgical 1 et de la portion de fil brute 10 selon au moins quatre rangées (4), orientées parallèlement à l'axe longitudinal A de la portion de fil chirurgical 1 et de la portion de fil brute 10 et disposées de façon régulière tout autour de l'axe longitudinal A de la portion de fil chirurgical 1 et de la portion de fil brute 10.

Comme représenté sur les exemples de réalisation des figures 1, 2 et 9, on entend par disposés de façon régulière que les axes de révolution des picots 3 de deux rangées 4 successives forment un angle aR, par exemple de 90° dans le cas où la portion de fil chirurgical 1 comporte quatre rangées 4 de picots 3.

De plus, cette disposition avantageuse permet également de faciliter l'obtention de la portion de fil brute 10 au cours de l'étape a), une telle portion de fil chirurgical 10 se démoulant aisément.

Enfin, cette disposition avantageuse permet d'obtenir une portion de fil chirurgical 1 munie de picots 3 répartis autour de sa partie de support 2 sans qu'une torsion de la portion de fil brute 10 ne soit nécessaire et comme enseigné par l'état de la technique (EP 2 690 206 A1), ce qui permet de simplifier et de réduire la durée du procédé selon l'invention.

Selon ce mode de réalisation de l'invention, le moule à picots M employé au cours de l'étape a) est conformé de telle sorte qu'il comporte au moins quatre parties (notamment identiques) M1, M2, M3, M4, ces parties M1, M2, M3, M4 étant séparées par des évents E, lorsque le moule M est fermé et mis sous pression, ces évents E étant disposés au niveau des plans de symétrie de la portion de fil brute 10 munie de picots 3, séparant chacun des picots 3 en deux parties symétriques par rapport à un de ces plans de symétrie, et débouchant dans les empreintes EP permettant la formation des picots 3.

Avantageusement, au moins deux évents E, sont formés par l'espace entre les deux coques du moule au niveau du plan de joint, lorsque le moule est fermé et mis sous pression.

Les évents E ont par exemple une largeur 1, lorsque le moule M est fermé et mis sous pression, notamment identique pour tous les évents E.

Ainsi lors du moulage au cours de l'étape a), une dépression va intervenir dans les évents E, faisant pénétrer partiellement le matériau polymère liquide dans ces évents E en plus des empreintes EP.

Cette disposition avantageuse permet de s'assurer que le polymère liquide pénètre bien dans l'intégralité de l'empreinte EP afin que chaque picot 3 soit correctement formé à l'issue de l'étape a).

On décrit ici également un fil chirurgical pour la reconstruction dans le domaine de la chirurgie, et/ou de la médecine esthétique comportant au moins une portion 1 réalisée par le procédé selon l'invention.

Un tel fil chirurgical, selon l'utilisation qui va en être faite, notamment selon le type de tissus avec lesquels il va coopérer, peut ainsi comporter par exemple une pluralité de portions avec chacune des caractéristiques propres, notamment au niveau des dimensions Lf, Df, et/ou des propriétés mécaniques, notamment la résistance à la rupture en traction, liées également éventuellement au matériau employé, de la partie de support 2 et/ou au niveau de la forme, des dimensions ou de la disposition (liée aux paramètres ef et af notamment) des picots 3 sur la partie de support 2.

### NOMENCLATURE

1. Portion de fil chirurgical
2. Partie de support
3. Picots
4. Rangée de picots
10. Portion de fil brute
A. Axe
Di. Diamètre initial
Df. Diamètre final
Ei. Ecartement initial
Ef. Ecartement final
ai. Inclinaison initiale
af. Inclinaison finale
Li. Longueur initiale
Lf. Longueur finale
Lp. Longueur de picot
Lb. Largeur de la base du picot
R. Rayon de courbure
20. Installation
21. Extrudeuse
23. Dispositif d'étirage
30. Installation
31. Dispositif de moulage par injection
31a. Système d'injection
31b. Réservoir d'alimentation
33. Dispositif d'étirage
M. Moule à picots
M1. Partie de moule
M2. Partie de moule
M3. Partie de moule
M4. Partie de moule
Ep. Empreinte à picot
E. Event
1. largeur des évents
G. Granulés

## Revendications

1. Procédé de fabrication d'une portion de fil chirurgical (1) pour la reconstruction dans le domaine de la chirurgie, et/ou de la médecine esthétique, ladite portion de fil chirurgical (1) comprenant une partie de support (2), en particulier de forme cylindrique, et une pluralité de picots (3) saillants de la partie de support (2) et répartis de façon régulière sur la partie de support (2), ladite portion de fil chirurgical (1) ayant une longueur (Lf), ledit procédé comprenant les étapes successives :
a). Obtention d'une portion de fil brute (10) de longueur (Li), avec Li<Lf, munie de picots (3) réalisés par moulage dans un moule à picots (M) comprenant une pluralité d'empreintes (EP) permettant la formation des picots (3),
b). Etirage de la portion de fil brute (10), la portion de fil chirurgical (1) ayant à l'issue de cette étape la longueur (Lf).
**caractérisé en ce que**, au cours de l'étape b), l'étirage de la portion de fil brute (10) est tel que sa longueur (Li) est multipliée au moins par 2.

2. Procédé selon la revendication 1, dans lequel :
- à l'issue de l'étape a), lesdits picots (3) sont inclinés d'un angle (ai) par rapport à l'axe longitudinal (A) de la portion de fil brute (10), étant compris entre 60° et 90°, de préférence entre 70° et 80°, et
- à l'issue de l'étape b), sous l'effet de l'étirage de la portion de fil brute (10), les picots (3) se retrouvent inclinés d'un angle (af) par rapport à l'axe longitudinal (A) de la portion de fil chirurgical (1), l'angle (af) étant strictement inférieur à l'angle (ai) et étant compris entre 30° et 60°, de préférence entre 40° et 50°.

3. Procédé selon l'une des revendications 1 ou 2, dans lequel la longueur (Lp) des picots (3) est supérieure au diamètre (Df) de la portion de fil chirurgical (1), et la largeur (Lb) de leur base, ménagée sur la partie de support (2) de la portion de fil chirurgical (1), est inférieure au diamètre (Df) de la portion de fil chirurgical (1).

4. Procédé de fabrication d'une portion de fil chirurgical (1) selon l'une des revendications 1 à 3, dans lequel l'étape a) d'obtention d'une portion de fil brute (10) comprend au moins trois sous-étapes a1), a2) et a3) :
a1). Formation par extrusion de la partie de support (2) de la portion de fil brute (10) à partir d'au moins un matériau polymère brut, à l'issue de laquelle la portion de fil brute (10) possède la longueur (Li),
a2). Introduction de la partie de support (2) de la portion de fil brute (10) dans un moule à picots (M),
a3). Mise sous pression de la partie de support (2) de la portion de fil brute (10) dans le moule à picots (M) afin de former les picots (3) saillants de la partie de support (2), lesdits picots (3) étant, à l'issue de cette étape, inclinés par rapport à l'axe longitudinal de l'angle (ai).

5. Procédé de fabrication d'une portion de fil chirurgical (1) selon la revendication 1 à 3, dans lequel l'étape a) d'obtention d'une portion de fil brute (10) comprend une unique sous-étape a1bis) de moulage par injection dans un moule à picots (M) de la partie de support (2) de la portion de fil brute (10) et des picots (3) saillants de la partie de support (2) à partir d'au moins un matériau polymère brute.

6. Procédé de fabrication d'une portion de fil chirurgical (1) selon l'une des revendications 1 à 5, dans lequel les picots (3) et la partie de support (2) sont réalisés dans le même matériau polymère.

7. Procédé de fabrication d'une portion de fil chirurgical selon l'une des revendications 1 à 6, dans lequel une étape c) de chauffage de la portion de fil brute (10) munie de picots (3) est prévue après l'étape a), et avant ou simultanément à l'étape b).

8. Procédé de fabrication d'une portion de fil chirurgical (1) selon l'une des revendications 1 à 7, dans lequel les picots (3) comportent une extrémité (P) de forme hémisphérique avec un rayon de courbure (R) supérieur à 0,1mm.

9. Procédé de fabrication d'une portion de fil chirurgical (1) selon l'une des revendications 1 à 8, dans lequel la portion de fil chirurgical (1) est résorbable et comprend au moins un matériau polymère résorbable.

10. Procédé de fabrication d'une portion de fil chirurgical (1) selon la revendication 9, dans lequel la portion de fil chirurgical (1) est constituée d'un mélange de PLLA et de PCL, notamment un mélange de 60% à 95% de PLLA et de 5% à 40% de PCL.

11. Procédé de fabrication d'une portion de fil chirurgical selon l'une des revendications 1 à 10, dans lequel les picots (3) sont répartis sur la partie de support (2) de la portion de fil chirurgical (1) et de la portion de fil brute (10) selon au moins quatre rangées (4), orientées parallèlement à l'axe longitudinal (A) de la portion de fil chirurgical (1) et de la portion de fil brute (10) et disposées de façon régulière tout autour de l'axe longitudinal (A) de la portion de fil chirurgical (1) et de la portion de fil brute (10).

12. Procédé de fabrication d'une portion de fil chirurgical selon la revendication 11, dans lequel le moule à picots (M) employé au cours de l'étape a) est conformé de telle sorte qu'il comporte au moins quatre parties (M1, M2, M3, M4), notamment identiques, les parties (M1, M2, M3, M4) étant séparées par des évents (E), lorsque le moule (M) est fermé et mis sous pression, ces évents (E) étant disposés au niveau des plans de symétrie de la portion de fil brute (10) munie de picots (3), séparant chacun des picots (3) en deux parties symétriques par rapport à un de ces plans de symétrie, et débouchant dans les empreintes (EP) permettant la formation des picots (3).

## Patentansprüche

1. Verfahren zur Herstellung eines Abschnitts eines chirurgischen Fadens (1) zur Rekonstruktion im Bereich der Chirurgie und/oder der ästhetischen Medizin, wobei der Abschnitt des chirurgischen Fadens (1) einen Trägerteil (2), insbesondere in zylindrischer Form, und eine Vielzahl von Widerhaken (3) umfasst, die aus dem Trägerteil (2) herausragen und auf regelmäßige Weise auf dem Trägerteil (2) verteilt sind, wobei der Abschnitt des chirurgischen Fadens (1) eine Länge (Lf) aufweist, wobei das Verfahren die aufeinanderfolgenden Schritte umfasst:
a). Erhalt eines Rohfadenabschnitts (10) der Länge (Li), wobei Li<Lf, versehen mit Widerhaken (3), die durch Formgießen in einer Widerhakenform (M) ausgeführt werden, die eine Vielzahl von Vertiefungen (EP) umfasst, die die Bildung der Widerhaken (3) erlauben,
b). Streckziehen des Rohfadenabschnitts (10), wobei der Abschnitt des chirurgischen Fadens (1) am Ende dieses Schritts die Länge (Lf) aufweist.
**dadurch gekennzeichnet, dass** im Verlauf von Schritt b) das Streckziehen des Rohfadenabschnitts (10) derart erfolgt, dass seine Länge (Li) mindestens verdoppelt wird.

2. Verfahren nach Anspruch 1, wobei:
- am Ende von Schritt a) die Widerhaken (3) in Bezug auf die Längsachse (A) des Rohfadenabschnitts (10) um einen Winkel (ai) geneigt sind, der zwischen 60° und 90°, vorzugsweise zwischen 70° und 80° liegt, und
- am Ende von Schritt b) die Widerhaken (3) unter der Wirkung des Streckziehens des Rohfadenabschnitts (10) um einen Winkel (af) in Bezug auf die Längsachse (A) des Abschnitts des chirurgischen Fadens (1) geneigt sind, wobei der Winkel (af) streng kleiner als der Winkel (ai) ist und zwischen 30° und 60°, vorzugsweise zwischen 40° und 50° liegt.

3. Verfahren nach einem der Ansprüche 1 oder 2, wobei die Länge (Lp) der Widerhaken (3) größer ist als der Durchmesser (Df) des Abschnitts des chirurgischen Fadens (1) und die Breite (Lb) ihrer Basis, die auf dem Trägerteil (2) des Abschnitts des chirurgischen Fadens (1) vorgesehen ist, kleiner ist als der Durchmesser (Df) des Abschnitts des chirurgischen Fadens (1).

4. Verfahren zur Herstellung eines Abschnitts eines chirurgischen Fadens (1) nach einem der Ansprüche 1 bis 3, wobei der Schritt a) zum Erhalt eines Rohfadenabschnitts (10) mindestens drei Teilschritte a1), a2) und a3) umfasst:
a1). Bildung des Trägerteils (2) des Rohfadenabschnitts (10) aus mindestens einem polymeren Rohmaterial durch Extrusion, an deren Ende der Rohfadenabschnitt (10) die Länge (Li) aufweist,
a2). Einführen des Trägerteils (2) des Rohfadenabschnitts (10) in eine Widerhakenform (M),
a3). Unterdrucksetzen des Trägerteils (2) des Rohfadenabschnitts (10) in der Widerhakenform (M), um die aus dem Trägerteil (2) vorstehenden Widerhaken (3) zu bilden, wobei die Widerhaken (3) am Ende dieses Schritts in Bezug auf die Längsachse um den Winkel (ai) geneigt sind.

5. Verfahren zur Herstellung eines Abschnitts eines chirurgischen Fadens (1) nach Anspruch 1 bis 3, wobei der Schritt a) des Erhalts eines Rohfadenabschnitts (10) einen einzigen Teilschritt a1bis) des Spritzgießens des Trägerteils (2) des Rohfadenabschnitts (10) und der aus dem Trägerteil (2) vorstehenden Widerhaken (3) aus mindestens einem polymeren Rohmaterial in eine Widerhakenform (M) umfasst.

6. Verfahren zur Herstellung eines Abschnitts eines chirurgischen Fadens (1) nach einem der Ansprüche 1 bis 5, wobei die Widerhaken (3) und der Trägerteil (2) aus dem gleichen Polymermaterial ausgeführt sind.

7. Verfahren zur Herstellung eines Abschnitts eines chirurgischen Fadens nach einem der Ansprüche 1 bis 6, wobei ein Schritt c) des Erwärmens des mit den Widerhaken (3) versehenen Rohfadenabschnitts (10) nach dem Schritt a) und vor oder gleichzeitig mit Schritt b) vorgesehen ist.

8. Verfahren zur Herstellung eines Abschnitts eines chirurgischen Fadens (1) nach einem der Ansprüche 1 bis 7, wobei die Widerhaken (3) ein Ende (P) in halbkugeliger Form mit einem Krümmungsradius (R) größer als 0,1 mm umfasst.

9. Verfahren zur Herstellung eines Abschnitts eines chirurgischen Fadens (1) nach einem der Ansprüche 1 bis 8, wobei der Abschnitt des chirurgischen Fadens (1) resorbierbar ist und mindestens ein resorbierbares Polymermaterial umfasst.

10. Verfahren zur Herstellung eines Abschnitts eines chirurgischen Fadens (1) nach Anspruch 9, wobei der Abschnitt des chirurgischen Fadens (1) aus einer Mischung aus PLLA und PCL, insbesondere einer Mischung aus 60 % bis 95 % PLLA und 5 % bis 40 % PCL besteht.

11. Verfahren zur Herstellung eines Abschnitts eines chirurgischen Fadens nach einem der Ansprüche 1 bis 10, wobei die Widerhaken (3) auf dem Trägerteil (2) des Abschnitts des chirurgischen Fadens (1) und des Rohfadenabschnitts (10) in mindestens vier Reihen (4) verteilt sind, die parallel zur Längsachse (A) des Abschnitts des chirurgischen Fadens (1) und des Rohfadenabschnitts (10) ausgerichtet und gleichmäßig um die Längsachse (A) des Abschnitts des chirurgischen Fadens (1) und des Rohfadenabschnitts (10) verteilt sind.

12. Verfahren zur Herstellung eines Abschnitts eines chirurgischen Fadens nach Anspruch 11, wobei die Widerhakenform (M), die in Schritt a) verwendet wird, derart angepasst ist, dass sie mindestens vier insbesondere identische Teile (M1, M2, M3, M4) umfasst, wobei die Teile (M1, M2, M3, M4) durch Entlüftungsöffnungen (E) getrennt sind, wenn die Form (M) geschlossen und unter Druck gesetzt ist, wobei diese Entlüftungsöffnungen (E) auf der Höhe der Symmetrieebenen des mit Widerhaken (3) versehenen Rohfadenabschnitts (10) angeordnet sind und jeden der Widerhaken (3) in zwei Teile trennen, die in Bezug auf eine dieser Symmetrieebenen symmetrisch sind, und in Vertiefungen (EP) münden, was die Bildung der Haken (3) ermöglicht.

## Claims

1. Method for the manufacture of a portion of surgical thread (1) for the purposes of reconstruction in the field of surgery, and/or cosmetic medicine, said portion of surgical thread (1) comprising a support part (2), in particular cylinder-shaped, and a plurality of barbs (3) protruding from the support part (2) and regularly distributed on the support part (2), said portion of surgical thread (1) having a length (Lf), said method comprising the successive steps of:
• a). Obtaining a raw thread portion (10) of length (Li), where Li<Lf, provided with barbs (3) produced by moulding in a barbed mould (M) comprising a plurality of cavities (EP) for forming the barbs (3),
• b). Stretching the raw thread portion (10) having, following this step, the length (Lf). **characterised in that**, during step b), the stretching of the raw thread portion (10) is such that the length (Li) thereof is multiplied by at least 2.

2. Method according to claim 1, wherein:
- following step a), said barbs (3) are inclined by an angle (ai) with respect to the longitudinal axis (A) of the raw thread portion (10), being between 60° and 90°, preferably between 70° and 80°, and
- following step b), under the effect of the stretching of the raw thread portion (10), the barbs (3) are inclined by an angle (af), with respect to the longitudinal axis (A) of the portion of surgical thread (1), the angle (af) being strictly less than the angle (ai) and being between 30° and 60°, preferably between 40° and 50°.

3. Method according to one of claims 1 or 2, wherein the length (Lp) of the barbs (3) is greater than the diameter (Df) of the portion of surgical thread (1), and the width (Lb) of the base thereof, formed on the support part (2) of the portion of surgical thread (1), is less than the diameter (Df) of the portion of surgical thread (1).

4. Method for the manufacture of a portion of surgical thread (1) according to one of claims 1 to 3, wherein step a) of obtaining a raw thread portion (10) comprises at least three substeps a1), a2), and a3):
a1). Forming by extrusion the support part (2) of the raw thread portion (10) using at least one raw polymer material, following which the raw thread portion (10) has the length (Li),
a2). Introducing the support part (2) of the raw thread portion (10) into a barbed mould (M),
a3). Compressing the support part (2) of the raw thread portion (10) in the barbed mould (M) so as to form the barbs (3) protruding from the support part (2), said barbs (3) being, following this step, inclined with respect to the longitudinal axis of the angle (ai).

5. Method for the manufacture of a portion of surgical thread (1) according to claims 1 to 3, wherein step a) of obtaining a raw thread portion (10) comprises a single substep (albis) of injection-moulding in a barbed mould (M) the support part (2) of the raw thread portion (10) and the barbs (3) protruding from the support part (2) using at least one raw polymer material.

6. Method for the manufacture of a portion of surgical thread (1) according to one of claims 1 to 5, wherein the barbs (3) and the support part (2) are made of the same polymer material.

7. Method for the manufacture of a portion of surgical thread according to one of claims 1 to 6, wherein a step c) of heating the raw thread portion (10) provided with barbs (3) is envisaged after step a), and before or simultaneously with step b).

8. Method for the manufacture of a portion of surgical thread (1) according to one of claims 1 to 7, wherein the barbs (3) include a hemispherical end (P) with a radius of curvature (R) greater than 0.1 mm.

9. Method for the manufacture of a portion of surgical thread (1) according to one of claims 1 to 8, wherein the portion of surgical thread (1) is resorbable and comprises at least one resorbable polymer material.

10. Method for the manufacture of a portion of surgical thread (1) according to claim 9, wherein the portion of surgical thread (1) consists of a mixture of PLLA and PCL, particularly a mixture of 60% to 95% PLLA and 5% to 40% PCL.

11. Method for the manufacture of a portion of surgical thread according to one of claims 1 to 10, wherein the barbs (3) are distributed along the support part (2) of the portion of surgical thread (1) and the raw thread portion (10) in at least four rows (4), oriented parallel with the longitudinal axis (A) of the portion of surgical thread (1) and the raw thread portion (10) and disposed regularly all around the longitudinal axis (A) of the portion of surgical thread (1) and the raw thread portion (10).

12. Method for the manufacture of a portion of surgical thread according to claim 11, wherein the barbed mould (M) used during step a) is shaped such that it includes at least four parts (M1, M2, M3, M4), particularly identical, the parts (M1, M2, M3, M4) being separated by vents (E), when the mould (M) is closed and compressed, these vents (E) being disposed at the level of the planes of symmetry of the raw thread portion (10) provided with barbs (3), separating each of the barbs (3) in two symmetrical parts with respect to one of these planes of symmetry, and opening into the cavities (EP) for forming the barbs (3).
